# EUROPEAN PATENT SPECIFICATION

(11) **EP 4 268 749 B1**
(45) Date of publication and mention of the grant of the patent: **20.05.2026**
(21) Application number: 23170310.9
(22) Date of filing: 27.04.2023
(51) Int. Cl.: A61B 18/14

(54) **SYSTEMS AND DEVICES FOR IMPROVED IRRIGATION FLOW DURING CARDIAC PROCEDURE**
SYSTEME UND VORRICHTUNGEN FÜR VERBESSERTEN IRRIGATIONSFLUSS WÄHREND EINES HERZEINGRIFFS
SYSTÈMES ET DISPOSITIFS POUR UN ÉCOULEMENT D'IRRIGATION AMÉLIORÉ PENDANT UNE PROCÉDURE CARDIAQUE

(30) Priority: 28.04.2022 US 202263336127 P; 28.03.2023 US 202318127600
(43) Date of publication of application: 01.11.2023
(73) Proprietor: BIOSENSE WEBSTER (ISRAEL) LTD., 2066717 Yokneam (IL)
(72) Inventor: OKARSKI, Kevin Mark, Irvine, 92618 (US); BAH, Abubakarr, Irvine, 92618 (US); NGUYEN, Thanh, Irvine, 92618 (US); DATTA, Keshava, Irvine, 92618 (US)
(74) Representative: Carpmaels & Ransford LLP

(56) References cited:
- WO-A1-2004/112629
- US-A- 5 928 228
- US-A1- 2012 271 138

## Description

### FIELD

The present invention relates generally to medical devices, and in particular catheters with electrodes and a spine retention hub having irrigation openings, and further relates to, but not exclusively, catheters suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art tend to utilize radiofrequency (RF) electrical energy to heat tissue. RF ablation can have certain drawbacks, such as heightened risk of thermal cell injury which can lead to tissue charring, burning, steam pop, phrenic nerve palsy, pulmonary vein stenosis, and esophageal fistula. Cryoablation is an alternative approach to RF ablation that generally reduces thermal risks associated with RF ablation. Maneuvering cryoablation devices and selectively applying cryoablation, however, is generally more challenging compared to RF ablation; therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. Nos. 2021/0169550A1, 2021/0169567A1, 2021/0169568A1, 2021/0161592A1, 2021/0196372A1, 2021/0177503A1, and 2021/01 86604A1.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Furthermore, multiple linear spines are generally assembled together by attaching both ends of the linear spines to a tubular shaft (e.g., a pusher tube) to form a spherical basket.

Due to the small size of the spines and the electrodes, however, adhering the electrodes to the spines and then forming a spherical basket from the multiple linear spines can be a difficult task, increasing the manufacturing time and cost and the chances that the electrode fails due to an improper bond or misalignment. Additionally, these cardiac procedures produce heat at the electrodes that could cause a patient harm. What is needed, therefore, are systems and devices for an improved spine retention hub with improved irrigation capabilities to effectively dissipate heat for a basket type catheter and medical probe with the spine retention hub and a basket assembly that can help to reduce the time required for manufacturing the basket assembly and alternative catheter geometries in general.
US2012271138A1 describes a system for sensing multiple local electric voltages from endocardial surface of a heart, including: an elongate tubular member; a plurality of flexible splines having proximal portions, distal portions and medial portions therein between; an anchor for securably affixing the proximal portions of the splines; a tip for securably affixing the distal portions of the splines; and a polymeric member including opposed a first open end and a second open end defining an open lumen therein between and an inner member surface and an outer member surface, wherein at least one of the plurality of flexible splines is at least partially disposed within the lumen of the polymeric member; a flexible electrode assembly strip with one or more exposed electrodes disposed on at least a portion of the outer surface of the polymeric member.

### SUMMARY

There is provided, in accordance with an embodiment of the present invention, a spine retention hub for a basket type catheter that may include a cylindrical member. The cylindrical member may include a first portion at a distal end of the spine retention hub having a first diameter, a second portion proximate the first portion and having a curved section with a first radius of curvature in a longitudinal direction where the first radius of curvature being approximately half the first diameter. The cylindrical member also may include a third portion proximate to the second portion at a proximal end of the spine retention hub and having a third diameter that is approximately three times the first diameter. The third portion third portion of the cylindrical member may include a plurality of relief lands and a plurality of brackets. Each bracket may be disposed at the proximal end of the spine retention hub and overlap a portion of a respective relief land of the plurality of relief lands. Additionally, each bracket may be, along with a respective relief land, configured to receive and retain a respective spine section. The cylindrical member may also include a plurality of first irrigation openings, each proximate the distal end of the spine retention hub.

The plurality of first irrigation openings may include six openings which may be aligned with the plurality of relief lands.

Each opening of the plurality of first openings may include a flow area of approximately 0.05 mm² to approximately 0.6 mm².

The spine retention hub may also include at least one electrode disposed on the first portion.

The at least one electrode may include a cylindrical electrode.

The at least one electrode may include a cylindrical shape that is truncated at two ends.

The plurality of brackets may be circumferentially spaced apart defining a plurality of gaps along the third portion of the cylindrical member. The plurality of brackets may be configured to receive and retain one or more wires.

The second portion of the cylindrical member may include a second diameter that transitions from the first diameter to the third diameter in the longitudinal direction about the curved section.

The curved section may include a concave section proximate the first portion and a convex section proximate the third portion.

Each bracket may include a curved bracket.

Each bracket may overlap a proximal end of a respective relief land.

Each bracket may be raised above an outer surface of the spine retention hub.

Each bracket may include a first cutout extending in the longitudinal direction and a radial direction of the spine retention hub.

Each bracket may include a second cutout that extends in the longitudinal direction and radial direction of the spine retention hub and separates each backet into two sections.

The third portion of the cylindrical member may include a plurality of elongated cavities configured to receive one or more wires.

The second portion of the cylindrical member may include a ring extending radially and may have a plurality of third cutouts configured to receive the one or more wires.

The plurality of third cutouts may be aligned about with the plurality of elongated cavities in the longitudinal direction.

Further described herein is a medical probe. The medical probe may include a spine retention hub including a cylindrical member. The cylindrical member may include a first portion at a distal end of the spine retention hub having a first diameter, a second portion proximate the first portion and having a curved section with a first radius of curvature in a longitudinal direction where the first radius of curvature being approximately half the first diameter. The cylindrical member also may include a third portion proximate to the second portion at a proximal end of the spine retention hub and having a third diameter that is approximately three times the first diameter. The third portion of the cylindrical member may include a plurality of relief lands and a plurality of brackets. Each bracket may be disposed at the proximal end of the spine retention hub and overlap a portion of a respective relief land of the plurality of relief lands. Additionally, each bracket may be, along with a respective relief land, configured to receive and retain a respective spine section. The cylindrical member may also include a plurality of first irrigation openings, each proximate the distal end of the spine retention hub. The medical probe may also include a flexible insertion tube that may have a proximal end and a distal end. The flexible insertion tube may extend along a longitudinal axis. The medical probe may also include an expandable basket assembly proximate the distal end of the flexible insertion tube. The expandable basket assembly may include a single unitary structure that may include the plurality of spine sections. The spine sections may converge at a central spine intersection, which may have one or more cutouts that may be configured to allow bending of the spine sections. Each spine section may have a respective end connected to the distal end of the flexible insertion tube with a respective bracket and the respective relief land. The central spine intersection may be positioned on the longitudinal axis at a distal end of the expandable basket assembly. The medical probe may also include one or more electrodes coupled to each of the spine sections. Each electrode may define a lumen through the electrode so that a spine section extends through the lumen of each of the one or more electrodes.

The plurality of spine sections may extend from the central spine intersection in an equiangular pattern such that respective angles between respectively adjacent spine sections are approximately equal.

The expandable basket assembly may include four to ten spine sections of the plurality of spine sections.

The expandable basket assembly may include six spine sections of the plurality of spine sections.

The expandable basket assembly may be approximately spherical.

The expandable basket assembly may be approximately oblate-spheroid.

Each lumen may be disposed offset with respect to a longitudinal axis of each electrode.

The expandable basket assembly may include at least one discrete cutout located proximate the central spine intersection.

The one or more cutouts may include a centrosymmetric pattern.

The one or more cutouts may include an equiangular pattern.

The one or more cutouts may extend along at least a portion of each spine section.

Each electrode may include a wire relief adjacent the lumen to allow for one or more wires to extend adjacent to the lumen.

The lumen may be disposed symmetrically about a longitudinal axis of the electrode.

The one or more electrodes may be configured to deliver electrical pulses for irreversible electroporation, the electrical pulses having a peak voltage of at least 900 volts (V).

The medical probe may also include a plurality of wires each electrically joined to a respective electrode of the one or more electrodes.

The plurality of spine sections may include nitinol.

The plurality of spine sections may include metallic strands.

The plurality of first irrigation openings may include six openings that may be aligned with the plurality of relief lands.

Each opening of the plurality of first openings may include a flow area of approximately 0.05 mm² to approximately 0.6 mm².

The medical probe may also include at least one electrode disposed on the first portion.

The at least one electrode may include a cylindrical electrode.

The at least one electrode may include a cylindrical shape that is truncated at two ends.

The plurality of brackets may be circumferentially spaced apart that may define a plurality of gaps along the third portion of the cylindrical member. The gaps may be configured to receive and retain one or more wires.

The first irrigation openings may be disposed on the second portion.

The second portion of the cylindrical member may have a second diameter that transitions from the first diameter to the third diameter about the curved section.

The curved section may include a concave section proximate the first portion and a convex section proximate the third portion.

Each bracket may include a curved bracket.

Each bracket may overlap a proximal end of a respective relief land.

Each bracket may be raised above an outer surface of the spine retention hub.

Each bracket may include a first cutout extending in the longitudinal direction and a radial direction of the spine retention hub.

Each bracket may include a second cutout that extends in the longitudinal direction and radial direction of the spine retention hub and separates each backet into two sections.

The third portion of the cylindrical member may include a plurality of elongated cavities configured to receive one or more wires.

The second portion of the cylindrical member may include a ring extending radially and having a plurality of third cutouts configured to receive the one or more wires.

The plurality of third cutouts may be aligned with the plurality of elongated cavities in the longitudinal direction.

Further described herein is a medical probe. The medical probe may include a spine retention hub including a cylindrical member. The cylindrical member may include a first portion at a distal end of the spine retention hub having a first diameter, a second portion proximate the first portion and having a curved section with a first radius of curvature in a longitudinal direction where the first radius of curvature being approximately half the first diameter. The cylindrical member also may include a third portion proximate to the second portion at a proximal end of the spine retention hub and having a third diameter that is approximately three times the first diameter. The third portion third portion of the cylindrical member may include a plurality of relief lands and a plurality of brackets. Each bracket may be disposed at the proximal end of the spine retention hub and overlap a portion of a respective relief land of the plurality of relief lands. Additionally, each bracket may be, along with a respective relief land, configured to receive and retain a respective spine section. The cylindrical member may also include a plurality of first irrigation openings, each proximate the distal end of the spine retention hub. The medical probe may also include a flexible insertion tube that may have a proximal end and a distal end. The flexible insertion tube may extend along a longitudinal axis. The medical probe may also include an expandable basket assembly proximate the distal end of the flexible insertion tube.

The expandable basket assembly may include a single unitary structure that may include the plurality of spine sections. The spine sections may converge at a central spine intersection, which may have one or more cutouts that may be configured to allow bending of the spine sections. Each spine section may have a respective end connected to the distal end of the flexible insertion tube with a respective bracket and the respective relief land. The central spine intersection may be positioned on the longitudinal axis at a distal end of the expandable basket assembly. The medical probe may also include one or more electrodes coupled to each of the spine sections. Each electrode may define a lumen through the electrode so that a spine section extends through the lumen of each of the one or more electrodes.

### BRIEF DESCRIPTION OF THE DRAWINGS

FIG. 1 is a schematic pictorial illustration of a medical system including a medical probe whose distal end includes a basket assembly with electrodes and a spine retention hub, in accordance with an embodiment of the present invention;
FIG. 2A is a schematic pictorial illustration showing a perspective view of a medical probe in an expanded form, in accordance with an embodiment of the present invention;
FIG. 2B is a schematic pictorial illustration showing a side view of a medical probe in a collapsed form, in accordance with embodiments of the present invention;
FIG. 2C is a schematic pictorial illustration showing an exploded side view of a medical probe, in accordance with an embodiment of the present invention;
FIG. 3 is a schematic pictorial illustration showing a profile outline of a basket assembly and spine retention hub of a given medical device, in accordance with an embodiment of the present invention; and
FIGS. 4A, 4B, 4C, 4D, 4E, and 4F are schematic pictorial illustrations showing provide outlines of spine retention hubs, in accordance with embodiments of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

The present disclosure is related to systems, methods or uses and devices which utilize end effectors having electrodes affixed to spines. Example systems, methods, and devices of the present disclosure may be particularly suited for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue, preferably by applying a pulsed electric field effective to induce electroporation in the myocardial tissue. The systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1.

To deliver pulsed field ablation (PFA) in an IRE (irreversible electroporation) procedure, electrodes should contact the tissue being ablated with a sufficiently large surface area. As described hereinbelow, the medical probe includes a tubular shaft having proximal and distal ends, and a basket assembly at the distal end of the tubular shaft. The basket assembly includes a single unitary structure. The unitary structure can include a plurality of linear spines formed from a planar sheet of material and one or more electrodes coupled to each of the spines. The plurality of linear spines can converge at a central spine intersection having one or more cutouts. The cutouts can allow for bending of each spine such that the spines form an approximately spherical or oblate-spheroid basket assembly.

Additionally, the spine retention hub may be configured to receive and retain the plurality of spines and include a plurality of irrigation openings that are configured to aim irrigation fluid (*e.g.,* saline solution) at the one or more electrodes found on the basket assembly of the medical probe and/or the ablation site of the patient in an efficient manner to quickly dissipate heat from the electrodes and/or the ablation site during the ablation process to minimize harm to the patient.

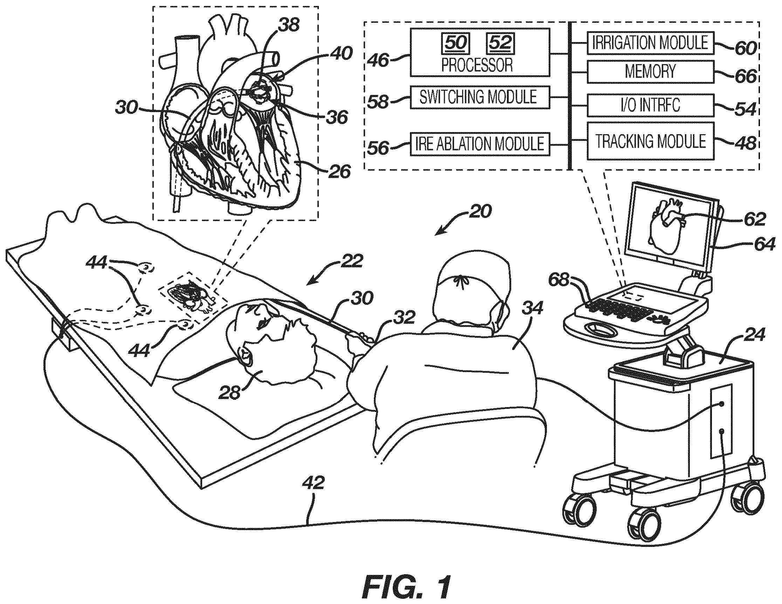

FIG. 1 is a schematic, pictorial illustration of a medical system 20 including a medical probe 22 and a control console 24, in accordance with an embodiment of the present invention. Medical system 20 may be based, for example, on the CARTO^{®} system, produced by Biosense Webster Inc. of 31 Technology Drive, Suite 200, Irvine, CA 92618 USA. In embodiments described hereinbelow, medical probe 22 can be used for diagnostic or therapeutic treatment, such as for performing ablation procedures in a heart 26 of a patient 28. Alternatively, medical probe 22 may be used, mutatis mutandis, for other therapeutic and/or diagnostic purposes in the heart or in other body organs.

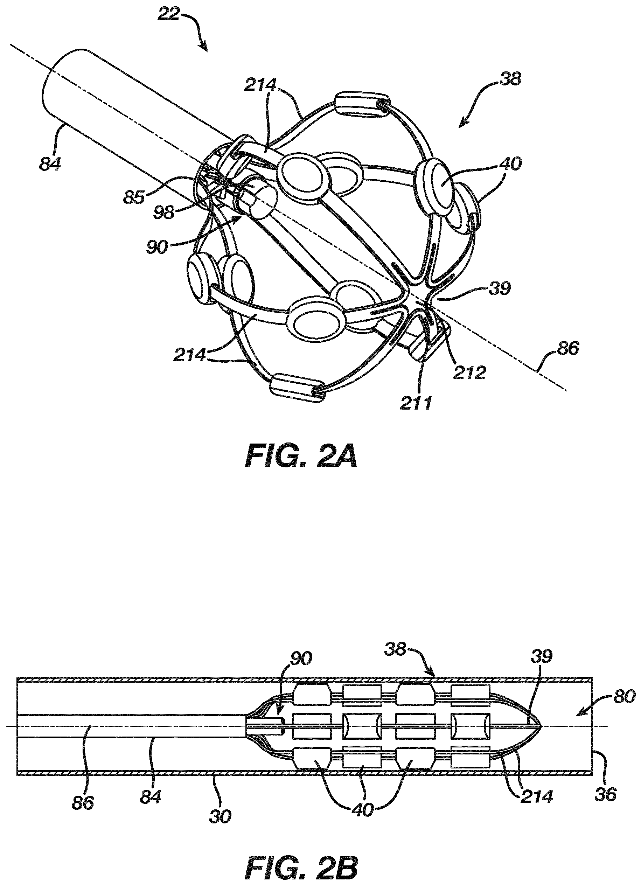

Medical probe 22 includes a flexible insertion tube 30 and a handle 32 coupled to a proximal end of the tubular shaft. During a medical procedure, a medical professional 34 can insert probe 22 through the vascular system of patient 28 so that a distal end 36 of the medical probe enters a body cavity such as a chamber of heart 26. Upon distal end 36 entering the chamber of heart 26, medical professional 34 can deploy a basket assembly 38 approximate a distal end 36 of the medical probe 22. Basket assembly 38 can include a plurality of electrodes 40 affixed to a plurality of spines 214, as described in the description referencing FIGS. 2A and 2B hereinbelow. To start performing a medical procedure such as irreversible electroporation (IRE) ablation, medical professional 34 can manipulate handle 32 to position distal end 36 so that electrodes 40 engage cardiac tissue at a desired location or locations. Upon positioning the distal end 36 so that electrodes 40 engages cardiac tissue, the medical professional 34 can activate the medical probe 22 such that electrical pulses are delivered by the electrodes 40 to perform the IRE ablation.

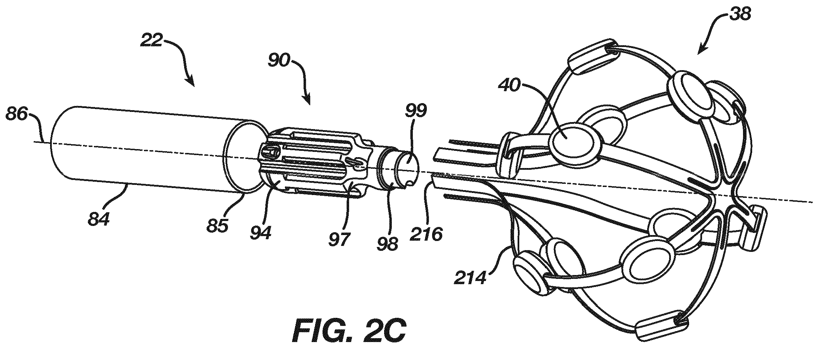

The medical probe 22 can include a guide sheath and a therapeutic catheter, wherein the guide sheath includes the flexible insertion tube 30 and the handle 32 and the therapeutic catheter includes the basket assembly 38, electrodes 40, and a tubular shaft 84 (see FIGS. 2A, 2B, and 2C). The therapeutic catheter is translated through the guide sheath so that the basket assembly 38 is positioned in the heart 26. The distal end 36 of the medical probe 22 corresponds to a distal end of the guide sheath when the basket assembly 38 is contained within the flexible insertion tube 30, and the distal end 36 of the medical probe 22 corresponds to a distal end of the basket assembly 38 when the basket assembly 38 is extended from the distal end of the guide sheath. The medical probe 22 can be alternatively configured to include a second handle on the therapeutic catheter and other features as understood by a person skilled in the pertinent art.

In the configuration shown in FIG. 1, control console 24 is connected, by a cable 42, to body surface electrodes, which typically include adhesive skin patches 44 that are affixed to patient 28. Control console 24 includes a processor 46 that, in conjunction with a tracking module 48, determines location coordinates of distal end 36 inside heart 26. Location coordinates can be determined based on electromagnetic position sensor output signals provided from the distal portion of the catheter when in the presence of a generated magnetic field. Location coordinates can additionally, or alternatively be based on impedances and/or currents measured between adhesive skin patches 44 and electrodes 40 that are affixed to basket assembly 38. In addition to being used as location sensors during a medical procedure, electrodes 40 may perform other tasks such as ablating tissue in the heart.

As described hereinabove, in conjunction with tracking module 48, processor 46 may determine location coordinates of distal end 36 inside heart 26 based on impedances and/or currents measured between adhesive skin patches 44 and electrodes 40. Such a determination is typically after a calibration process relating the impedances or currents to known locations of the distal end has been performed. While embodiments presented herein describe electrodes 40 that are preferably configured to deliver IRE ablation energy to tissue in heart 26, configuring electrodes 40 to deliver any other type of ablation energy to tissue in any body cavity is considered to be within the spirit and scope of the present invention. Furthermore, although described in the context of being electrodes 40 that are configured to deliver IRE ablation energy to tissue in the heart 26, one skilled in the art will appreciate that the disclosed technology can be applicable to electrodes used for mapping and/or determining various characteristics of an organ or other part of the patient's 28 body.

Processor 46 may include real-time noise reduction circuitry 50 typically configured as a field programmable gate array (FPGA), followed by an analog-to-digital (A/D) signal conversion integrated circuit 52. The processor can be programmed to perform one or more algorithms and uses circuitry 50 and circuit 52 as well as features of modules to enable the medical professional 34 to perform the IRE ablation procedure.

Control console 24 also includes an input/output (I/O) communications interface 54 that enables control console 24 to transfer signals from, and/or transfer signals to electrodes 40 and adhesive skin patches 44. In the configuration shown in FIG. 1, control console 24 additionally includes an IRE ablation module 56 and a switching module 58.

IRE ablation module 56 is configured to generate IRE pulses having peak power in the range of tens of kilowatts. In some examples, the electrodes 40 are configured to deliver electrical pulses having a peak voltage of at least 900 volts (V). The medical system 20 performs IRE ablation by delivering IRE pulses to electrodes 40. Preferably, the medical system 20 delivers biphasic pulses between electrodes 40 on the spine. Additionally, or alternatively, the medical system 20 delivers monophasic pulses between at least one of the electrodes 40 and a skin patch.

In order to dissipate the heat and to improve the efficiency of the ablation process, system 20 supplies irrigation fluid (e.g., a saline solution) to distal end 36 and to the electrodes 40 via a channel (not shown) in tubular shaft 84 (see FIGS. 2A through 2C) and via irrigation openings 98 of the spine retention hub 90 (*see* FIGS. 2C, 3, and 4A-D). Additionally, or alternatively, irrigation fluid can be supplied through the flexible insertion tube 30 to irrigation openings 98 of the spine retention hub 90. Control console 24 includes an irrigation module 60 to monitor and control irrigation parameters, such as the pressure and the temperature of the irrigation fluid.

Based on signals received from electrodes 40 and/or adhesive skin patches 44, processor 46 can generate an electroanatomical map 62 that shows the location of distal end 36 in the patient's body. During the procedure, processor 46 can present map 62 to medical professional 34 on a display 64, and store data representing the electroanatomical map in a memory 66. Memory 66 may include any suitable volatile and/or non-volatile memory, such as random-access memory or a hard disk drive.

In some embodiments, medical professional 34 can manipulate map 62 using one or more input devices 68. In alternative embodiments, display 64 may include a touchscreen that can be configured to accept inputs from medical professional 34, in addition to presenting map 62.

FIG. 2A is a schematic pictorial illustration showing a perspective view of a medical probe 22 having a basket assembly 38 in an expanded form when unconstrained, such as by being advanced out of an insertion tube lumen 80 at a distal end 36 of an insertion tube 30. The medical probe 22 illustrated in FIG. 2A lacks the guide sheath illustrated in FIG. 1. FIG. 2B shows the basket assembly in a collapsed form within insertion tube 30 of the guide sheath. In the expanded form (FIG. 2A), spines 214 bow radially outwardly and in the collapsed form (FIG. 2B) the spines are arranged generally along a longitudinal axis 86 of insertion tube 30.

As shown in FIG. 2A, basket assembly 38 includes a plurality of flexible spines 214 that are formed at the end of a tubular shaft 84 and are connected at both ends. During a medical procedure, medical professional 34 can deploy basket assembly 38 by extending tubular shaft 84 from insertion tube 30 causing basket assembly 38 to exit insertion tube 30 and transition to the expanded form. Spines 214 may have elliptical (e.g., circular) or rectangular (that may appear to be flat) cross-sections, and include a flexible, resilient material (e.g., a shape-memory alloy such as nickel-titanium, also known as Nitinol) forming a strut as will be described in greater detail herein.

As shown in FIG. 2A, the plurality of flexible linear spines 214 converge at a central spine intersection 211. In some examples central spine intersection 211 can include one or more cutouts 212 that allow for bending of the spines 214 when each spine respective attachment end 216 is connected to the spine retention hub 90, described in more detail below.

In embodiments described herein, one or more electrodes 40 positioned on spines 214 of basket assembly 38 can be configured to deliver ablation energy (RF and/or IRE) to tissue in heart 26. Additionally, or alternatively, the electrodes can also be used to determine the location of basket assembly 38 and/or to measure a physiological property such as local surface electrical potentials at respective locations on tissue in heart 26. The electrodes 40 can be biased such that a greater portion of the one or more electrodes 40 face outwardly from basket assembly 38 such that the one or more electrodes 40 deliver a greater amount of electrical energy outwardly away from the basket assembly 38 (i.e., toward the heart 26 tissue) than inwardly.

Examples of materials ideally suited for forming electrodes 40 include gold, platinum and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 26.

Basket assembly 38 has a distal end 39. The medical probe 22 can include a spine retention hub 90 that extends longitudinally from a distal end of tubular shaft 84 towards distal end 39 of basket assembly 38. As described supra, control console 24 includes irrigation module 60 that delivers irrigation fluid to basket assembly 38 through tubular shaft 84.

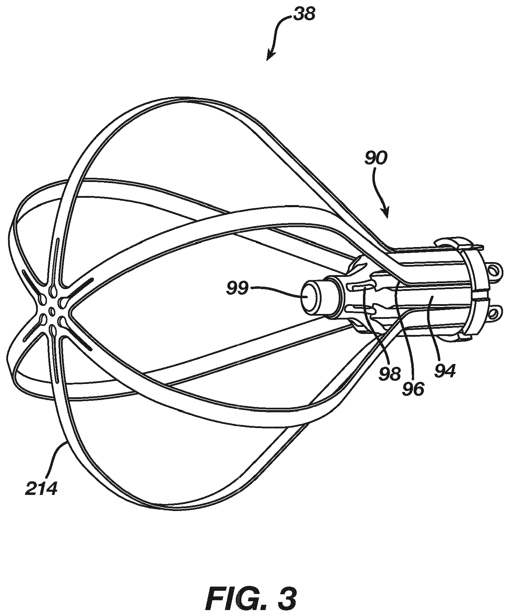

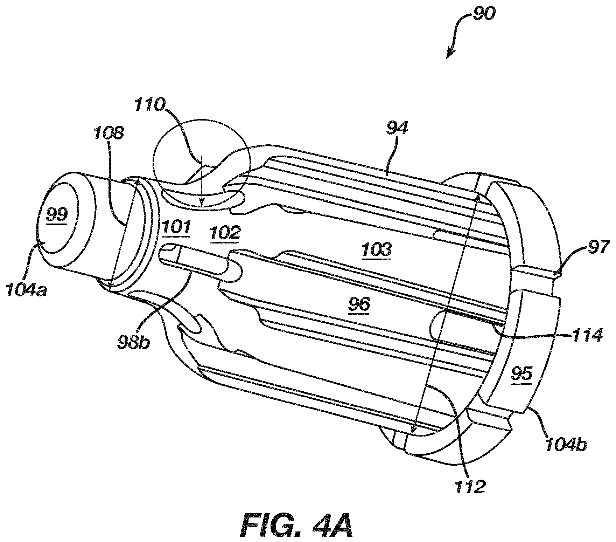

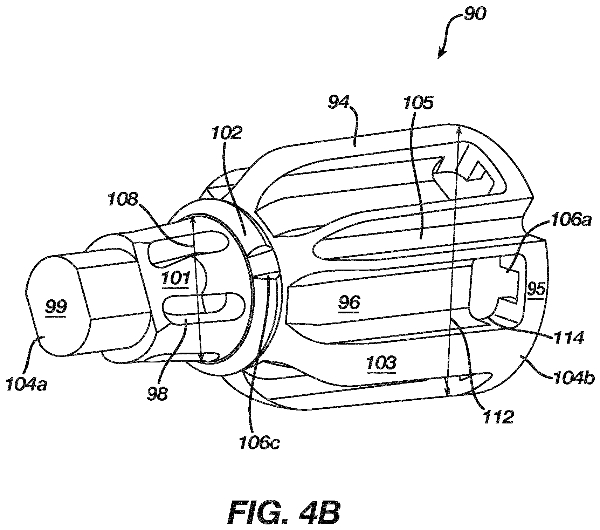

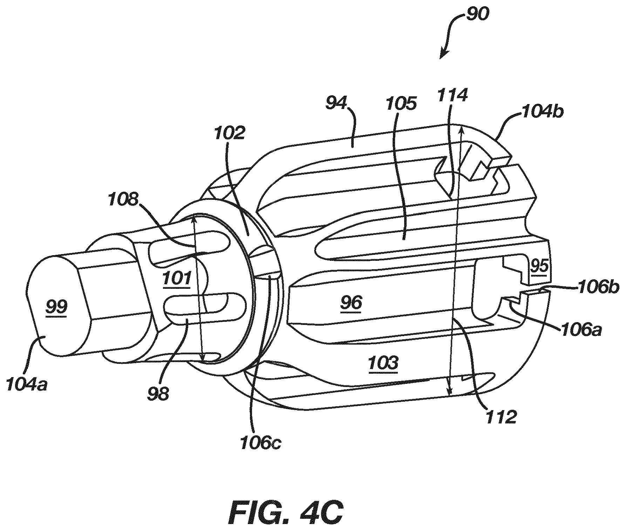

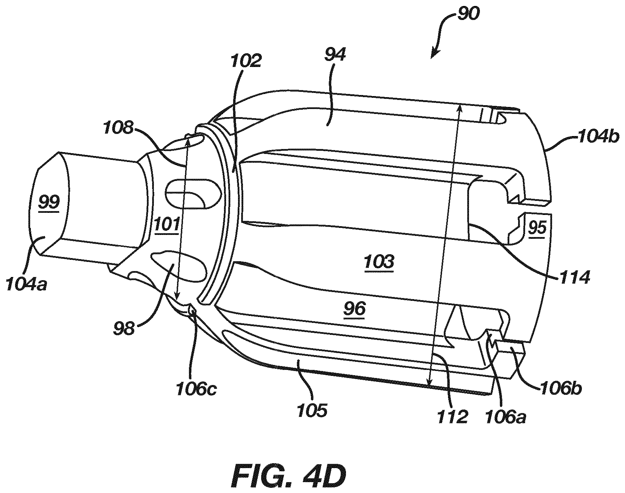

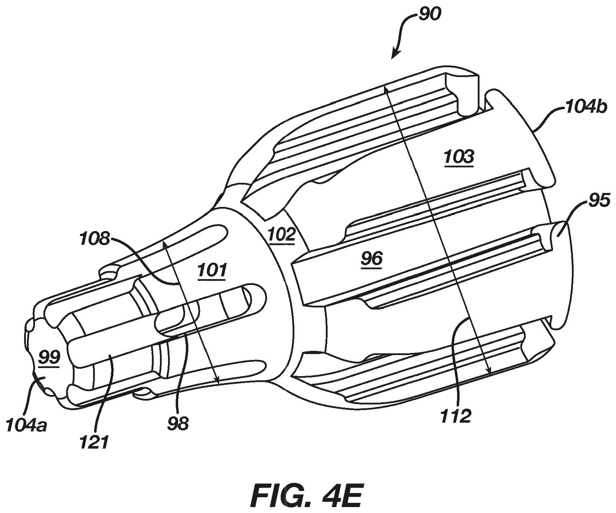

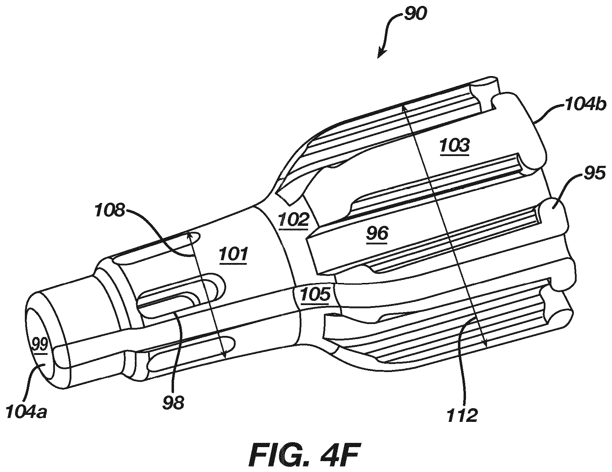

Turning to FIG. 2C and FIG. 3, basket assembly 38 includes a single unitary structure that includes a plurality of linear spines 214 formed from a planar sheet of material 210 or a tubular material. The spine retention hub 90 (described more in FIGS. 4A-4D) can be inserted into the tubular shaft 84 and attached to the tubular shaft 84. Spine retention hub 90 can include a cylindrical member 94 including a plurality of relief lands 96, multiple irrigation openings 98, and at least one spine retention hub electrode 99, or some combination thereof. The spine retention hub electrode 99 may include a cylindrical shape *(see e.g.,* FIGS. 4A, 4E, and 4F) which may or may not be truncated at two opposing ends *(see e.g.,* FIGS. 4B, 4C, and 4D). Relief lands 96 can be disposed on the outer surface and/or a third portion 103 (described below with respect to FIGS. 4B, 4C, and 4D) of cylindrical member 94 and configured to allow a portion of each spine 214, such as each spine attachment end 216, to be fitted into a respective relief land 96. The attachment end 216 can be a generally linear end of the spine 214. The attachment end 216 can be configured to extend outwardly from the spine retention hub 90 such that the basket assembly 38 is positioned outwardly from the spine retention hub 90 and, consequently, outwardly from the tubular shaft 84. In this way, the spine 214 can be configured to position the basket assembly 38 distally from the distal end of the tubular shaft 84 and distal from the distal end of the insertion tube 30 when the basket assembly is deployed.

As described supra, control console 24 includes irrigation module 60 that delivers irrigation fluid to distal end 36. The multiple irrigation openings 98 can be angled to spray or otherwise disperse of the irrigation fluid to either a given electrode 40 or to tissue in heart 26. Since electrodes 40 do not include irrigation openings that deliver irrigation fluid, the configuration described hereinabove enables heat to be transferred from the tissue (i.e., during an ablation procedure) to the portion of the electrodes on the inner side of the spines 214, and the electrodes 40 can be cooled by aiming the irrigation fluid, via irrigation openings 98, at the portion of the electrodes 40 on the inner side of the spines 214. Spine retention hub electrode 99 disposed at a distal end of retention hub 90 can be used in combination with electrodes 40 on the spines 214, or alternatively, can be used independently from electrodes 40 for reference mapping or ablation.

FIGS. 4A, 4B, 4C, 4D, 4E, and 4F show various spine retention hubs 90 according with embodiments of the present invention. FIG. 4A shows a spine retention hub 90 that may include a cylindrical member 94. The cylindrical member 94 may include a first portion 101 at a distal end 104a of the spine retention hub 90 having a first diameter 108.

The cylindrical member 94 may also have a second portion 102 proximate the first portion 101 that has a curved section with a first radius of curvature 110 in a longitudinal direction. The first radius of curvature 110 may be approximately half the first diameter 108. The second portion 102 may also have a second diameter that transitions from the first diameter 108 to the third diameter 112 of the third portion 103 in the longitudinal direction about the curved section of the second portion 102. The curved section of the second portion 102 may include a concave section proximate the first portion 101 and a convex section proximate the third portion 103. The second portion 102 may include a ring extending radially and having a plurality of third cutouts 106c configured to receive one or more wires (not shown). The plurality of third cutouts 106c may be aligned with the plurality of elongated cavities 105 in the longitudinal direction. In some embodiments (see e.g., FIG. 4F) the spine retention hub may include an elongated cavity 105 that extends from the proximal end 104b to a distal end 104a through the first, second, and third portions 101, 102, and 103 of the spine retention hub 90.

The cylindrical member 94 may also include a third portion 103 proximate to the second portion 102 at a proximal end 104b of the spine retention hub 90. The third portion 103 may have a third diameter 112 that is approximately three times the first diameter 108. The third portion 103 may include a plurality of relief lands 96, a plurality of elongated cavities 105 configured to receive one or more wires, and a plurality of brackets 95. Each bracket 95 may be disposed at the proximal end 104b of the spine retention hub 90 and each bracket 95 may be overlapping a portion of a respective relief land 96 of the plurality of relief lands 96. In some embodiments (*see e.g.,* FIGS. 4E and 4F, each of the plurality of relief lands 06 may include stepped walls connecting an upper surface of the third portion 103 with the cavity floor of the relief land 96.

Each bracket 95 may be, along with a respective relief land 96, configured to receive and retain a respective spine section 214 and may be curved in the circumferential direction about the third portion 103. Each bracket 95 may overlap a proximal end of a respective relief land 96 as well as its relief land opening 114. The plurality of brackets 95 may be circumferentially spaced apart defining a plurality of gaps 97 (*see* FIG. 4A) along the third portion of the cylindrical member 94 that are configured to receive and retain one or more wires (not shown). Each bracket 95 may be raised above an outer surface of the spine retention hub 90 *(see e.g.,* FIGS. 4A-4E). In some cases, each bracket 95 may include a first cutout 106a extending in the longitudinal direction and a radial direction of the spine retention hub 90 *(see e.g.,* FIGS. 4B-4E) and/or a second cutout extending in the longitudinal direction and radial direction and separating each bracket 95 into two sections *(see e.g.,* FIGS. 4C-4E).

The cylindrical member 94 may also include a plurality of irrigation openings 98. Each irrigation opening may be proximate the distal end 104a of the spine retention hub 90. Also, the cylindrical member 94 may include a plurality of relief land openings 114, each of which may be proximate the proximate end 104b of the spine retention hub 90. The plurality irrigation openings 98 may include six openings 98 that are aligned with the plurality of relief lands 96. Each opening 98 may include a flow area of approximately 0.05 mm² to approximately 0.6 mm² (e.g., approximately 0.24 mm²). As described above, the plurality of irrigations openings 98 are configured to aim irrigation fluid (e.g., saline solution) at the one or more electrodes 40 found on the basket assembly 38. At least one irrigation opening 98 may be configured to aim (or selectively aim) irrigation fluid at an ablation site of the patient as well. In some embodiment, (e.g., FIG. 4E), the irrigation opening is adjacent to a groove 121 configured to further assist in aiming the irrigation fluid.

The embodiments described above are cited by way of example, and the present invention is not limited by what has been particularly shown and described hereinabove. Rather, the scope of the invention is defined by the appended claims.

## Claims

1. A spine retention hub (90) for a basket type catheter, comprising:
a cylindrical member (94) comprising:
a first portion (101) at a distal end (104a) of the spine retention hub having a first diameter (108); a second portion (102) proximate the first portion and having a curved section with a first radius of curvature (110) in a longitudinal direction, the first radius of curvature being approximately half the first diameter; and
a third portion (103) proximate to the second portion at a proximal end (104b) of the spine retention hub and having a third diameter (112) that is approximately three times the first diameter, the third portion comprising:
a plurality of relief lands (96);
a plurality of brackets (95), each disposed at the proximal end of the spine retention hub, each overlapping a portion of a respective relief land of the plurality of relief lands, wherein each bracket is, along with a respective relief land, configured to receive and retain a respective spine section (214); and
a plurality of first irrigation openings (98), each proximate the distal end of the spine retention hub.

2. The spine retention hub according to claim 1, wherein the plurality of first irrigation openings comprises six openings (98) which are aligned with the plurality of relief lands.

3. The spine retention hub according to claim 1, further comprising at least one electrode disposed on the first portion.

4. The spine retention hub according to claim 3, wherein the at least one electrode comprises a cylindrical electrode, or a cylindrical shape that is truncated at two ends.

5. The spine retention hub according to claim 1, wherein the plurality of brackets are circumferentially spaced apart defining a plurality of gaps (97) along the third portion of the cylindrical member that are configured to receive and retain one or more wires.

6. The spine retention hub according to claim 1, wherein the second portion of the cylindrical member has a second diameter that transitions from the first diameter to the third diameter in the longitudinal direction about the curved section.

7. The spine retention hub according to claim 6, wherein the curved section comprises a concave section proximate the first portion and a convex section proximate the third portion.

8. The spine retention hub according to claim 7, wherein each bracket comprises a curved bracket and overlaps a proximal end of a respective relief land.

9. The spine retention hub according to claim 8, wherein each bracket is raised above an outer surface of the spine retention hub.

10. The spine retention hub according to claim 9, wherein each bracket comprises a first cutout (106a) extending in the longitudinal direction and a radial direction of the spine retention hub.

11. The spine retention hub according to claim 10, wherein each bracket comprises a second cutout that extends in the longitudinal direction and radial direction of the spine retention hub and separates each bracket into two sections.

12. The spine retention hub according to claim 11, wherein:
the third portion of the cylindrical member comprises a plurality of elongated cavities (105) configured to receive one or more wires, and
the second portion of the cylindrical member comprises a ring extending radially and having a plurality of third cutouts (106c) configured to receive the one or more wires.

13. The spine retention hub according to claim 12, wherein the plurality of third cutouts are aligned about with the plurality of elongated cavities in the longitudinal direction.

14. A medical probe (22), comprising:
the spine retention hub according to claim 1;
a flexible insertion tube (30) having a proximal end and a distal end (36), the flexible insertion tube extending along a longitudinal axis (86);
an expandable basket assembly (38) proximate the distal end of the flexible insertion tube, the expandable basket assembly comprising:
a single unitary structure that comprises the plurality of spine sections, the spine sections converging at a central spine intersection, the central spine intersection (211) having one or more cutouts (212) that are configured to allow bending of the spine sections, each spine section having a respective end connected to the distal end of the flexible insertion tube with a respective bracket and the respective relief land, the central spine intersection being positioned on the longitudinal axis at a distal end of the expandable basket assembly; and
one or more electrodes (40) coupled to each of the spine sections, each electrode defining a lumen through the electrode so that a spine section extends through the lumen of each of the one or more electrodes.

15. The spine retention hub according to claim 1 or the medical probe according to claim 14, wherein each opening of the plurality of first irrigation openings comprises a flow area of approximately 0.05 mm² to approximately 0.6 mm².

16. The medical probe according to claim 14, wherein the plurality of spine sections extend from the central spine intersection in an equiangular pattern such that respective angles between respectively adjacent spine sections are approximately equal.

17. A medical probe (22), comprising:
the spine retention hub according to claim 1;
a flexible insertion tube (30) having a proximal end and a distal end (36), the flexible insertion tube extending along a longitudinal axis (86); and
an expandable basket assembly (38) proximate the distal end of the flexible insertion tube.

18. The medical probe according to claim 17, wherein the expandable basket assembly comprises:
a single unitary structure that comprises the plurality of spine sections, the spine sections converging at a central spine intersection, the central spine intersection (211) having one or more cutouts (212) that allows for bending of the spine sections, each spine section having a respective end connected to the distal end of the flexible insertion tube with a respective curved bracket and the respective relief land, the central spine intersection being positioned on the longitudinal axis at a distal end of the expandable basket assembly; and
one or more electrodes (40) coupled to each of the spine sections, each electrode defining a lumen through the electrode so that a spine section extends through the lumen of each of the one or more electrodes.

## Patentansprüche

1. Strebenrückhaltenabe (90) für einen Korb-Typ-Katheter, umfassend:
ein zylindrisches Element (94), umfassend:
einen ersten Abschnitt (101) an einem distalen Ende (104a) der Strebenrückhaltenabe, der einen ersten Durchmesser (108) aufweist; einen zweiten Abschnitt (102) in der Nähe des ersten Abschnitts, der einen gekrümmten Abschnitt mit einem ersten Krümmungsradius (110) in Längsrichtung aufweist, wobei der erste Krümmungsradius etwa die Hälfte des ersten Durchmessers beträgt; und
einen dritten Abschnitt (103) in der Nähe des zweiten Abschnitts an einem proximalen Ende (104b) der Strebenrückhaltenabe und aufweisend einen dritten Durchmesser (112), der etwa das Dreifache des ersten Durchmessers beträgt, der dritte Abschnitt umfassend:
eine Vielzahl von Entlastungsflächen (96);
eine Vielzahl von Klammern (95), die jeweils am proximalen Ende der Strebenrückhaltenabe angeordnet sind und jeweils einen Abschnitt einer entsprechenden Entlastungsfläche der Vielzahl von Entlastungsflächen überlappen, wobei jede Klammer gemeinsam mit einer entsprechenden Entlastungsfläche konfiguriert ist, um einen entsprechenden Strebenabschnitt (214) aufzunehmen und zu halten; und
eine Vielzahl von ersten Durchflussöffnungen (98), die sich jeweils in der Nähe des distalen Endes der Strebenrückhaltenabe befinden.

2. Strebenrückhaltenabe nach Anspruch 1, wobei die Vielzahl der ersten Durchflussöffnungen sechs Öffnungen (98) umfasst, die auf die Vielzahl der Entlastungsflächen ausgerichtet sind.

3. Strebenrückhaltenabe nach Anspruch 1, ferner umfassend mindestens eine auf dem ersten Abschnitt angeordnete Elektrode.

4. Strebenrückhaltenabe nach Anspruch 3, wobei die mindestens eine Elektrode eine zylindrische Elektrode oder eine zylindrische Form, die an zwei Enden abgeschnitten ist, umfasst.

5. Strebenrückhaltenabe nach Anspruch 1, wobei die Vielzahl von Klammern in Umfangsrichtung voneinander beabstandet sind und eine Vielzahl von Lücken (97) entlang des dritten Abschnitts des zylindrischen Elements definieren, die konfiguriert sind, um einen oder mehrere Drähte aufzunehmen und zu halten.

6. Strebenrückhaltenabe nach Anspruch 1, wobei der zweite Abschnitt des zylindrischen Elements einen zweiten Durchmesser aufweist, der in der Längsrichtung um den gekrümmten Abschnitt vom ersten Durchmesser zum dritten Durchmesser übergeht.

7. Strebenrückhaltenabe nach Anspruch 6, wobei der gekrümmte Abschnitt einen konkaven Abschnitt in der Nähe des ersten Abschnitts und einen konvexen Abschnitt in der Nähe des dritten Abschnitts umfasst.

8. Strebenrückhaltenabe nach Anspruch 7, wobei jede Klammer eine gebogene Klammer umfasst und ein proximales Ende einer entsprechenden Entlastungsfläche überlappt.

9. Strebenrückhaltenabe nach Anspruch 8, wobei jede Halterung über eine Außenoberfläche der Strebenrückhaltenabe angehoben ist.

10. Strebenrückhaltenabe nach Anspruch 9, wobei jede Halterung einen ersten Ausschnitt (106a) umfasst, der sich in der Längsrichtung und einer radialen Richtung der Strebenrückhaltenabe erstreckt.

11. Strebenrückhaltenabe nach Anspruch 10, wobei jede Halterung einen zweiten Ausschnitt umfasst, der sich in der Längsrichtung und radialen Richtung der Strebenrückhaltenabe erstreckt und jede Halterung in zwei Abschnitte trennt.

12. Strebenrückhaltenabe nach Anspruch 11, wobei:
der dritte Abschnitt des zylindrischen Elements eine Vielzahl von länglichen Hohlräumen (105) umfasst, die konfiguriert sind, um einen oder mehrere Drähte aufzunehmen, und
der zweite Abschnitt des zylindrischen Elements einen Ring umfasst, der sich radial erstreckt und eine Vielzahl von dritten Ausschnitten (106c) aufweist, die konfiguriert sind, um den einen oder die mehreren Drähte aufzunehmen.

13. Strebenrückhaltenabe nach Anspruch 12, wobei die Vielzahl von dritten Ausschnitten etwa mit der Vielzahl von länglichen Hohlräumen in der Längsrichtung ausgerichtet sind.

14. Medizinische Sonde (22), umfassend:
die Strebenrückhaltenabe nach Anspruch 1;
ein flexibles Einführrohr (30), das ein proximales Ende und ein distales Ende (36) aufweist, wobei sich das flexible Einführrohr entlang einer Längsachse (86) erstreckt;
eine expandierbare Korbanordnung (38) nahe dem distalen Ende des flexiblen Einführrohrs, die expandierbare Korbanordnung umfassend:
eine einzige einheitliche Struktur, die eine Vielzahl von Strebenabschnitten umfasst, wobei die Strebenabschnitte an einem zentralen Strebenschnittpunkt zusammenlaufen, wobei der zentrale Strebenschnittpunkt (211) einen oder mehrere Ausschnitte (212) aufweist, die konfiguriert sind, um ein Biegen der Strebenabschnitte zu ermöglichen, wobei jeder Strebenabschnitt ein jeweiliges Ende aufweist, das mit dem distalen Ende des flexiblen Einführrohrs mit einer jeweiligen Halterung und der jeweiligen Entlastungsfläche verbunden ist, wobei der zentrale Strebenschnittpunkt auf der Längsachse an einem distalen Ende der expandierbaren Korbanordnung angeordnet ist; und
eine oder mehrere Elektroden (40), die mit jedem der Strebenabschnitte gekoppelt sind, wobei jede Elektrode ein Lumen durch die Elektrode definiert, so dass sich ein Strebenabschnitt durch das Lumen jeder der einen oder der mehreren Elektroden erstreckt.

15. Strebenrückhaltenabe nach Anspruch 1 oder medizinische Sonde nach Anspruch 14, wobei jede Öffnung der Vielzahl von ersten Durchflussöffnungen eine Durchflussfläche von etwa 0,05 mm² bis etwa 0,6 mm² umfasst.

16. Medizinische Sonde nach Anspruch 14, wobei sich die Vielzahl von linearen Strebenabschnitten von dem zentralen Strebenschnittpunkt in einem gleichwinkligen Muster derart erstrecken, dass die jeweiligen Winkel zwischen jeweils benachbarten Strebenabschnitten etwa gleich sind.

17. Medizinische Sonde (22), umfassend:
die Strebenrückhaltenabe nach Anspruch 1;
ein flexibles Einführrohr (30), das ein proximales Ende und ein distales Ende (36) aufweist, wobei sich das flexible Einführrohr entlang einer Längsachse (86) erstreckt; und
eine expandierbare Korbanordnung (38) nahe dem distalen Ende des flexiblen Einführrohrs.

18. Medizinische Sonde nach Anspruch 17, wobei die expandierbare Korbanordnung umfasst:
eine einzige einheitliche Struktur, die eine Vielzahl von Strebenabschnitten umfasst, wobei die Strebenabschnitte an einem zentralen Strebenschnittpunkt zusammenlaufen, wobei der zentrale Strebenschnittpunkt (211) einen oder mehrere Ausschnitte (212) aufweist, die ein Biegen der Strebenabschnitte ermöglichen, wobei jeder Strebenabschnitt ein jeweiliges Ende aufweist, das mit dem distalen Ende des flexiblen Einführrohrs mit einer jeweiligen gebogenen Halterung und der jeweiligen Entlastungsfläche verbunden ist, wobei der zentrale Strebenschnittpunkt auf der Längsachse an einem distalen Ende der expandierbaren Korbanordnung angeordnet ist; und
eine oder mehrere Elektroden (40), die mit jedem der Strebenabschnitte gekoppelt sind, wobei jede Elektrode ein Lumen durch die Elektrode definiert, so dass sich ein Strebenabschnitt durch das Lumen jeder der einen oder der mehreren Elektroden erstreckt.

## Revendications

1. Noix de rétention d'éléments d'armature (90) destinée à un cathéter de type panier, comprenant :
un élément cylindrique (94) comprenant :
une première partie (101) au niveau d'une extrémité distale (104a) de la noix de rétention d'éléments d'armature ayant un premier diamètre (108) ; une deuxième partie (102) à proximité de la première partie et ayant une section courbée avec un premier rayon de courbure (110) dans une direction longitudinale, le premier rayon de courbure valant approximativement la moitié du premier diamètre ; et
une troisième partie (103) à proximité de la deuxième partie au niveau d'une extrémité proximale (104b) de la noix de rétention d'éléments d'armature et ayant un troisième diamètre (112) qui vaut approximativement trois fois le premier diamètre, la troisième partie comprenant :
une pluralité de zones de soulagement (96) ;
une pluralité de supports (95), disposés chacun au niveau de l'extrémité proximale de la noix de rétention d'éléments d'armature, chevauchant chacun une partie d'une zone de soulagement respective de la pluralité de zones de soulagement, dans laquelle chaque support est, en même temps qu'une zone de soulagement respective, conçu pour recevoir et retenir une section d'élément d'armature (214) respective ; et
une pluralité de premières ouvertures d'irrigation (98), à proximité chacune de l'extrémité distale de la noix de rétention d'éléments d'armature.

2. Noix de rétention d'éléments d'armature selon la revendication 1, dans laquelle la pluralité de premières ouvertures d'irrigation comprend six ouvertures (98) qui sont alignées avec la pluralité de zones de soulagement.

3. Noix de rétention d'éléments d'armature selon la revendication 1, comprenant en outre au moins une électrode disposée sur la première partie.

4. Noix de rétention d'éléments d'armature selon la revendication 3, dans laquelle l'au moins une électrode comprend une électrode cylindrique, ou une forme cylindrique qui est tronquée au niveau de deux extrémités.

5. Noix de rétention d'éléments d'armature selon la revendication 1, dans laquelle la pluralité de supports sont espacés circonférentiellement définissant une pluralité d'espaces (97) le long de la troisième partie de l'élément cylindrique qui sont conçus pour recevoir et retenir un ou plusieurs fils.

6. Noix de rétention d'éléments d'armature selon la revendication 1, dans laquelle la deuxième partie de l'élément cylindrique a un deuxième diamètre qui passe du premier diamètre au troisième diamètre dans la direction longitudinale autour de la section courbée.

7. Noix de rétention d'éléments d'armature selon la revendication 6, dans laquelle la section courbée comprend une section concave à proximité de la première partie et une section convexe à proximité de la troisième partie.

8. Noix de rétention d'éléments d'armature selon la revendication 7, dans laquelle chaque support comprend un support courbé et chevauche une extrémité proximale d'une zone de soulagement respective.

9. Noix de rétention d'éléments d'armature selon la revendication 8, dans laquelle chaque support est surélevé au-dessus d'une surface externe de la noix de rétention d'éléments d'armature.

10. Noix de rétention d'éléments d'armature selon la revendication 9, dans laquelle chaque support comprend une première découpe (106a) s'étendant dans la direction longitudinale et une direction radiale de la noix de rétention d'éléments d'armature.

11. Noix de rétention d'éléments d'armature selon la revendication 10, dans laquelle chaque support comprend une deuxième découpe qui s'étend dans la direction longitudinale et la direction radiale de la noix de rétention d'éléments d'armature et sépare chaque support en deux sections.

12. Noix de rétention d'éléments d'armature selon la revendication 11, dans laquelle :
la troisième partie de l'élément cylindrique comprend une pluralité de cavités allongées (105) conçues pour recevoir un ou plusieurs fils, et
la deuxième partie de l'élément cylindrique comprend une bague s'étendant radialement et ayant une pluralité de troisièmes découpes (106c) conçues pour recevoir le ou les fils.

13. Noix de rétention d'éléments d'armature selon la revendication 12, dans laquelle la pluralité de troisièmes découpes sont alignées autour avec la pluralité de cavités allongées dans la direction longitudinale.

14. Sonde médicale (22), comprenant :
la noix de rétention d'éléments d'armature selon la revendication 1 ;
un tube d'insertion flexible (30) ayant une extrémité proximale et une extrémité distale (36), le tube d'insertion flexible s'étendant le long d'un axe longitudinal (86) ;
un ensemble panier expansible (38) à proximité de l'extrémité distale du tube d'insertion flexible, l'ensemble panier expansible comprenant :
une structure d'un seul tenant qui comprend la pluralité de sections d'élément d'armature, les sections d'élément d'armature convergeant au niveau d'une intersection centrale d'éléments d'armature, l'intersection centrale d'éléments d'armature (211) ayant une ou plusieurs découpes (212) qui sont conçues pour permettre un cintrage des sections d'élément d'armature, chaque section d'élément d'armature ayant une extrémité respective reliée à l'extrémité distale du tube d'insertion flexible avec un support respectif et la zone de soulagement respective, l'intersection centrale d'éléments d'armature étant positionnée sur l'axe longitudinal au niveau d'une extrémité distale de l'ensemble panier expansible ; et
une ou plusieurs électrodes (40) accouplées à chacune des sections d'élément d'armature, chaque électrode définissant une lumière à travers l'électrode de sorte qu'une section d'élément d'armature s'étend à travers la lumière de chacune parmi la ou les électrodes.

15. Noix de rétention d'éléments d'armature selon la revendication 1 ou sonde médicale selon la revendication 14, dans laquelle chaque ouverture de la pluralité de premières ouvertures d'irrigation comprend une aire d'écoulement d'approximativement 0,05 mm² à approximativement 0,6 mm².

16. Sonde médicale selon la revendication 14, dans laquelle la pluralité de sections d'élément d'armature s'étendent à partir de l'intersection centrale d'éléments d'armature dans un motif équiangulaire de telle sorte que des angles respectifs entre des sections d'élément d'armature respectivement adjacentes sont approximativement égaux.

17. Sonde médicale (22), comprenant :
la noix de rétention d'éléments d'armature selon la revendication 1 ;
un tube d'insertion flexible (30) ayant une extrémité proximale et une extrémité distale (36), le tube d'insertion flexible s'étendant le long d'un axe longitudinal (86) ; et
un ensemble panier expansible (38) à proximité de l'extrémité distale du tube d'insertion flexible.

18. Sonde médicale selon la revendication 17, dans laquelle l'ensemble panier expansible comprend :
une structure d'un seul tenant qui comprend la pluralité de sections d'élément d'armature, les sections d'élément d'armature convergeant au niveau d'une intersection centrale d'éléments d'armature, l'intersection centrale d'éléments d'armature (211) ayant une ou plusieurs découpes (212) qui permettent un cintrage des sections d'élément d'armature, chaque section d'élément d'armature ayant une extrémité respective reliée à l'extrémité distale du tube d'insertion flexible avec un support courbé respectif et la zone de soulagement respective, l'intersection centrale d'éléments d'armature étant positionnée sur l'axe longitudinal au niveau d'une extrémité distale de l'ensemble panier expansible ; et
une ou plusieurs électrodes (40) accouplées à chacune des sections d'élément d'armature, chaque électrode définissant une lumière à travers l'électrode de sorte qu'une section d'élément d'armature s'étend à travers la lumière de chacune parmi la ou les électrodes.
